(19) Europäisches Patentamt
European Patent Office
Office européen des brevets

(11) Veröffentlichungsnummer: **0 086 945**
**B1**

(12) # EUROPÄISCHE PATENTSCHRIFT

(45) Veröffentlichungstag der Patentschrift: **05.12.84**

(21) Anmeldenummer: **83100328.0**

(22) Anmeldetag: **15.01.83**

(51) Int. Cl.³: **C 07 C 33/03,** C 07 C 29/40, A 61 K 7/46, C 11 B 9/00, A 23 L 1/226

(54) **Neue Alkenole(I), Verfahren zu deren Herstellung, Verwendung von (I) als Riechstoffe und Riechstoffkompositionen mit einem Gehalt an (I).**

(30) Priorität: **27.01.82 CH 491/82**

(43) Veröffentlichungstag der Anmeldung: **31.08.83 Patentblatt 83/35**

(45) Bekanntmachung des Hinweises auf die Patenterteilung: **05.12.84 Patentblatt 84/49**

(84) Benannte Vertragsstaaten: **CH DE FR GB LI NL**

(56) Entgegenhaltungen: **EP - A - 0 045 453**

(73) Patentinhaber: **L. GIVAUDAN & CIE Société Anonyme, CH-1214 Vernier-Genève (CH)**

(72) Erfinder: **Kaiser, Roman, Weidstrasse 6, CH-8610 Uster (CH)**
Erfinder: **Lamparsky, Dietmar, Dr., Sonnhalde 8, CH-8602 Wangen (CH)**

(74) Vertreter: **Urech, Peter, Dr. et al, Grenzacherstrasse 124 Postfach 3255, CH-4002 Basel (CH)**

**EP 0 086 945 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft neue Riechstoffe. Es handelt sich dabei um die Verbindungen der Formel:

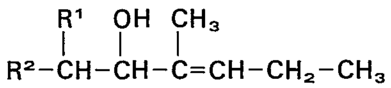

(I)

worin $R^1$ für Wasserstoff und $R^2$ für geradkettiges oder endständig einmal verzweigtes $C_{5-7}$-Alkyl oder $C_{4-7}$-Alkenyl steht, oder $R^1$ Methyl und $R^2$ geradkettiges oder endständig einmal verzweigtes $C_{4-7}$-Alkyl ist.

Beispiele von $C_{4-7}$- bzw. $C_{5-7}$-Alkylresten sind n-Butyl, iso-Butyl, n-Amyl, iso-Amyl, n-Hexyl, iso-Hexyl, n-Heptyl, iso-Heptyl. Bevorzugte Reste sind n-Amyl und iso-Amyl (insbesondere im Falle von $R^1$ = H) und n-Butyl bzw. iso-Butyl (insbesondere im Falle von $R^1$ = $CH_3$).

Beispiele von $C_{4-7}$-Alkenylresten sind Methallyl, Butenyl, Methylbutenyl, Methylpentenyl, Pentenyl etc. Die endständig verzweigten Alkenyle wie z.B. Methylbutenyl oder Methylpentenyl sind bevorzugt.

Eine bevorzugte Untergruppe von Verbindungen I bildet diejenige mit $R^1$ = Wasserstoff und $R^2$ = geradkettiges oder endständig einmal verzweigtes $C_{5-7}$-Alkyl oder $C_{4-7}$-Alkenyl. Die gesättigten Reste $R^2$ wiederum sind bevorzugt.

Die Formel soll demgemäss insbesondere die sekundären Alkohole:

| | |
|---|---|
| 4-Methylundec-3-en-5-ol | (Ia)* |
| 4-Methyldodec-3-en-5-ol | (Ib) |
| 4-Methyltridec-3-en-5-ol | (Ic) |
| 4,6-Dimethyldec-3-en-5-ol | (Id)* |
| 4,6-Dimethylundec-3-en-5-ol | (Ie) |
| 4,9-Dimethyldec-3-en-5-ol | (If)* |
| 4,6,8-Trimethylnon-3-en-5-ol | (Ig)* |
| 2,6-Dimethyl-1,6-nonadien-5-ol | (Ih)* |
| 4-Methyl-3,9-decadien-5-ol | (Ii) |
| 4-Methyl-3,8-(Z)-undecadien-5-ol | (Ij) |
| 4,9-Dimethyl-3,8-decadien-5-ol | (Ik) |
| 4,10-Dimethyl-3,9-undecadien-5-ol | (Il) |

in Form ihrer jeweils möglichen Stereoisomeren (cis- bzw. trans-Konfiguration an den Doppelbindungen) umfassen. Die mit einem Stern markierten Verbindungen sind bevorzugt.

Die Erfindung betrifft ferner ein Verfahren zur Herstellung der Verbindungen der Formel I.

Dieses Verfahren ist dadurch gekennzeichnet, dass man 2-Methyl-2-pentenal mit einer Verbindung der Formel:

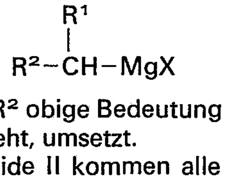

(II)

worin $R^1$ und $R^2$ obige Bedeutung besitzen und X für Halogen steht, umsetzt.

Als Halogenide II kommen alle Halogenide in Frage, doch wird vorzugsweise das Bromid verwendet.

Die Umsetzung des Methylpentenals mit der Verbindung II erfolgt zweckmässigerweise nach den an sich bekannten Methoden der Grignard-Reaktion, siehe z.B. „Organikum, Org. Chem. Grundpraktikum", Nachdruck 15. Aufl., VEB deutscher Verlag der Wissenschaften, Berlin 1977, 617 seq. Man arbeitet also zweckmässigerweise in Diäthyläter oder einem höheren Alkyläther bzw. in Tetrahydrofuran als Lösungsmittel und bei Temperaturen von ca. 0 bis 80° C.

Die Verbindungen I weisen besondere organoleptische Eigenschaften auf, auf Grund derer sie sich vorzüglich als Riechstoffe eignen.

Die Erfindung betrifft demgemäss auch die Verwendung der Verbindung I als Riechstoffe.

Das 4-Methylundec-3-en-5-ol Ia beispielsweise besitzt einen blumigen, fruchtigen und zugleich grün wirkenden Geruch, der im Fond durch pudrige, an Schokolade erinnernde Nuancen ergänzt wird, während das 4,6,8-Trimethylnon-3-en-5-ol Ig in erster Linie fruchtige, beerenartige und zusätzlich angenehm würzige Geruchsnoten besitzt. Im Falle der $C_{4-7}$-Alkenylderivate weisen die Produkte zumeist noch eine überraschende zusätzliche fettig-butterartige Geruchsnote auf.

Die Verbindungen der Formel I eignen sich aufgrund ihrer natürlichen Geruchsnoten insbesondere zur Modifizierung von bekannten, z.B.

a) blumigen Kompositionen (z.B. für Colognetypen u. ä., Extraits, Seifen und Kosmetika), wo insbesondere die blumigen Noten intensiviert werden,

b) des weiteren aber auch von Chypre- und Fougèretypen, die durch Zusatz von Verbindungen I moderner, belebter wirken und insbesondere einen sehr angenehmen frisch-grünen Aspekt erhalten (Extraittypen und Eaux de Cologne der maskulinen Richtung).

Die Verbindungen I verbinden sich mit zahlreichen bekannten Riechstoffingredienzien natürlichen oder synthetischen Ursprungs, wobei die Palette der natürlichen Rohstoffe sowohl leicht- als auch mittel- und schwerflüchtige Komponenten, und diejenige der Synthetika Vertreter aus praktisch allen Stoffklassen umfassen kann, wie dies aus der folgenden Zusammenstellung ersichtlich ist:

*Naturprodukte:* Basilikumöl, Baummoos-Absolue, Bergamottöl, Cassisknospen-Absolue, Zedernholzöl, *Ciste labdanum*, Korianderöl, Eichenmoos-Absolue, Eleminöl, Fichtennadelöl, Galbanum, Geraniumöl, Jasmin-Absolue und sein synthetischer Ersatz, Jonquille-Absolue, Lavendelöl, Mandarinenöl, Mastix-Absolue, Palmarosaöl, Patchouliöl, Petitgrainöl Paraguay, Sandelholzöl, Weihrauch, Ylang-Ylang-Öl und sein synthetischer Ersatz, Zitronenöl etc.

*Alkohole:* Citronellol, Geraniol, cis-3-Hexenol, Linalool, Sandela® (3-Isocamphyl-5-cyclohexanol), 2,2,8-Trimethyl-7-nonen-3-ol etc.

*Aldehyde:* α-Amylzimtaldehyd, Cyclamenaldehyd, Hydroxycitronellal, 2,6,10-Trimethylundec-9-en-1-al(Adoxal®) etc.

*Ketone:* 3,7,7-Trimethyl-3-[3'-methyl-2'-butenyl]bicyclo-[4.1.0]-hepten-4-on, α-Jonon, Vertofix® ( = acetyliertes Zedernholzöl) etc.

*Ester:* Amylsalicylat, Benzylacetat, Citronellylacetat, cis-3-Hexenylacetat, 1-Methyl-2-sec-butyl-

cyclohexylacetat, Methyldihydrojasmonat, Phenyläthylisobutyrat, Phenyläthyltiglat, 1,3,6,6-Tetramethylcyclohex-2-encarbonsäureäthylester, 3,6,6-Trimethyl-2-äthylcyclohex-2-encarbonsäureäthylester etc.

*Verschiedene:* Eugenol, Limonen, p-Menthan-8-thiol-3-on, 1-Methylcyclododecylmethyläther, γ-Undecalacton, Ambrettemoschus, Galaxolid® (1,3,4,6,7,8-Hexahydro-4,6,6,7,8,8-Hexamethylcyclopenta-γ-2-benzopyran), Ketonmoschus, Musk 174® (12-Oxahexadecanolid) etc.

Die Verbindungen der Formel I lassen sich in weiten Grenzen einsetzen, die beispielsweise von 0,1 (Detergenzien) bis 25% (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen, z.B. mit bis zu 40%, neuartige Komplexe aufbauen kann. Die bevorzugten Konzentrationen bewegen sich zwischen 0,5 und 20%. Die mit I hergestellten Kompositionen lassen sich für alle Arten von parfümierten Verbrauchsgütern einsetzen (Eaux de Cologne, Eaux de Toilette, Extraits, Lotionen, Cremes, Shampoos, Seifen, Salben, Puder, Zahnpasten, Mundwässer, Desodorants, Detergenzien, Tabak etc.)

Die Verbindungen I können demgemäss bei der Herstellung von Kompositionen und – wie obige Zusammenstellung zeigt – unter Verwendung einer breiten Palette bekannter Riechstoffe verwendet werden. Bei der Herstellung solcher Kompositionen können die oben aufgeführten bekannten Riechstoffe nach (dem Parfümeur bekannter) Art und Weise verwendet werden, wie z.B. aus W.A. Poucher, „Perfumes, Cosmetics and Soaps", *2*, 7. Aufl., Chapman und Hall, London, 1974, hervorgehen.

*Beispiel 1:*

In einer für Grignard-Reaktionen üblichen Apparatur werden 14,30 g (0,59 g-Atome) Magnesium in 100 ml Äther vorgelegt. Unter Rühren und unter Schutzgasatmosphäre ($N_2$) werden anschliessend 99,0 g (0,60 mol) 1-Bromhexan in 400 ml absolutem Äther so zugetropft, dass der Äther nach Einsetzen der Reaktion ständig schwach siedet. Nach beendeter Zugabe wird noch 30 min bei Rückflusstemperatur gehalten, dann auf 10° C abgekühlt und eine Lösung von 49,1 g (0,50 mol) 2-Methyl-2-pentenal in 300 ml Äther während 30 min so zugetropft, dass die Reaktionstemperatur ständig zwischen 10 und 25° C liegt. Zwecks Beendigung der Reaktion wird noch 1 h bei Rückflusstemperatur gehalten, dann der Grignard-Komplex mit gesättigter Ammoniumchloridlösung und Eis zersetzt, die überstehende ätherische Lösung mit gesättigter Kochsalzlösung gewaschen und getrocknet. Nach Abdampfen des Lösungsmittels verbleiben 105,3 g Rohprodukt, die fraktioniert destilliert werden. Man erhält so 77,8 g (84,4%) olfaktisch gutes 4-Methyl-3-undecen-5-ol vom Siedepunkt 66° C/0,04 mmHg.

Spektrale Daten: IR: 3340, 2958, 2924, 2858, 1670, 1460, 1378, 1303, 1045, 1002, 854 $cm^{-1}$.
MS: 184($M^+$, 7), 169(2), 155(26), 113(5), 99(100), 81(26), 71(15), 55(17), 43(70), 41(18).
Geruch: blumig, fruchtig, grün, an Schokolade erinnernd.

*Beispiel 2:*

Analog Beispiel 1 wird das Grignard-Reagens, welches durch Reaktion von 3,38 g (0,139 g-Atome) Magnesium in 20 ml Äther und 24,89 g (0,139 mol) 1-Bromheptan in 80 ml Äther erhältlich ist, mit 11,90 g (0,12 mol) 2-Methyl-2-pentenal in 30 ml Äther umgesetzt.

Die fraktionierte Destillation des Rohproduktes (29,5 g) ergibt 16,3 g (68,5%) olfaktisch gutes 4-Methyl-3-dodecen-5-ol vom Siedepunkt 74° C/0,04 mmHg.

Spektrale Daten: IR: 3340, 2920, 2850, 1670, 1460, 1375, 1300, 1048, 1010, 852 $cm^{-1}$.
MS: 198($M^+$, 19), 169(25), 127(7), 109(2), 99(100), 81(17), 69(5), 55(8), 43(20), 41(14).
Geruch: grün, blumig, fruchtig.

*Beispiel 3:*

Analog Beispiel 1 wird das Grignard-Reagens, welches durch Reaktion von 3,38 g (0,139 g-Atome) Magnesium in 20 ml Äther und 26,9 g (0,139 mol) 1-Bromoctan in 80 ml Äther erhältlich ist, mit 11,80 g (0,12 mol) 2-Methyl-2-penten-1-al in 30 ml Äther umgesetzt.

Die fraktionierte Destillation des Rohproduktes (28,9 g) ergibt 20,7 g (81,2%) olfaktisch gutes 4-Methyl-3-tridecen-5-ol vom Siedepunkt 85° C/0,15 mmHg.

Spektrale Daten: IR: 3340, 2920, 2842, 1670, 1458, 1377, 1302, 1112, 1070, 995, 852 $cm^{-1}$.
MS: 212($M^+$, 3), 183(12), 141(3), 99(87), 81(24), 71(18), 57(15), 55(22), 43(100), 41(30).
Geruch: fruchtig, grün, fettig.

*Beispiel 4:*

Analog Beispiel 1 wird das Grignard-Reagens, welches durch Reaktion von 8,03 g (0,33 g-Atome) Magnesium in 100 ml Äther und 58,18 g (0,35 mol) 2-Bromhexan in 250 ml Äther erhältlich ist, mit 28,3 g (0,29 mol) 2-Methyl-2-pentenal in 50 ml Äther umgesetzt.

Die fraktionierte Destillation des Rohproduktes (43,0 g) ergibt 26,4 g (49,4%) olfaktisch gutes 4,6-Dimethyl-3-decen-5-ol vom Siedepunkt 105° C/12 mmHg.

Spektrale Daten: IR: 3380, 2958, 2924, 2865, 2855, 1670, 1460, 1378, 1300, 1000, 850 $cm^{-1}$.
MS: 184($M^+$, 1), 155(1), 109(1), 99(60), 85(3), 81(15), 71(10), 55(18), 43(100), 41(38).
Geruch: grün, fettig, blumig, fruchtig, an Schokolade erinnernde Nuancen.

*Beispiel 5:*

Analog Beispiel 1 wird das Grignard-Reagens, welches durch Reaktion von 3,38 g (0,139 g-Atome) Magnesium in 20 ml Äther und 24,89 g

(0,139 mol) 2-Bromheptan in 80 ml Äther erhältlich ist mit 11,90 g (0,12 mol) 2-Methyl-2-pentenal in 30 ml Äther umgesetzt.

Die fraktionierte Destillation des Rohproduktes (28,7 g) ergibt 12,1 g (50,9%) olfaktisch gutes 4,6-Dimethyl-3-undecen-5-ol vom Siedepunkt 65° C/0,04 mmHg.

Spektrale Daten: IR: 3380, 2958, 2920, 2860, 1670, 1458, 1385, 1300, 1000, 850 $cm^{-1}$.

MS: 198($M^+$, 6), 169(37), 99(100), 81(5), 71(4), 55(7), 43(17), 41(15).

Geruch: grün, blumig, fruchtig (Beeren).

*Beispiel 6:*

Analog Beispiel 1 wird das Grignard-Reagens, welches durch Reaktion von 3,38 g (0,139 g-Atome) Magnesium in 20 ml Äther und 23,0 g (0,139 mol) 1-Brom-4-methylpentan in 80 ml Äther erhältlich ist, mit 11,9 g (0,12 mol) 2-Methyl-2-pentenal in 30 ml Äther umgesetzt.

Die fraktionierte Destillation des Rohproduktes (23,2 g) ergibt 17,2 g (77,8%) olfaktisch gutes 2,7-Dimethyl-7-decen-6-ol (bzw. 4,9-Dimethyldec-3-en-5-ol) vom Siedepunkt 81° C/ 0,1 mmHg.

Spektrale Daten: IR: 3340, 2950, 2922, 2862, 1670, 1460, 1385, 1365, 1303, 1068, 1045, 1010, 854 $cm^{-1}$.

MS: 184(5), 155(13), 109(3), 99(100), 85(11), 81(32), 71(15), 69(17), 55(18), 43(77), 41(20).

Geruch: grün, fruchtig, blumig, an Schokolade erinnernde Nuancen.

*Beispiel 7:*

Analog Beispiel 1 wird das Grignard-Reagens, welches durch Reaktion von 19,2 g (0,79 g-Atome) Magnesium in 200 ml Äther und 169,9 g (0,80 mol) 2-Brom-4-methylpentan in 400 ml Äther erhältlich ist, mit 68,0 g (0,69 mol) 2-Methyl-2-pentenal in 200 ml Äther umgesetzt.

Die fraktionierte Destillation des Rohproduktes (137 g) ergibt 54,0 g (42,5%) olfaktisch gutes 2,4,6-Trimethyl-6-nonen-5-ol (bzw. 4,6,8-Trimethylnon-3-en-5-ol) vom Siedepunkt 100°C/12 mmHg.

Spektrale Daten: IR: 3400, 2955, 2922, 2863, 1670, 1460, 1384, 1366, 1065, 1005, 960, 853 $cm^{-1}$.

MS: 184($M^+$, <0,2), 109(1), 99(71), 85(5), 81(17), 71(11), 57(7), 55(15), 43(100), 41(22).

Geruch: grün, fruchtig, nach Beeren, würzig.

*Beispiel 8:*

Analog Beispiel 1 wird das Grignard-Reagens, welches durch Reaktion von 6,60 g (0,27 g-Atome) Magnesium in 30 ml Tetrahydrofuran und 28,50 g (0,27 mol) 3-Methyl-3-buten-1-ylchlorid in 120 ml Tetrahydrofuran erhältlich ist, mit 22,90 g (0,23 mol) 2-Methyl-2-pentenal in 40 ml Tetrahydrofuran umgesetzt.

Die fraktionierte Destillation des Rohproduktes (46,6 g) ergibt 24,1 g (62,3%) olfaktisch gutes 2,6-Dimethyl-1,6-nonadien-5-ol vom Siedepunkt 62° C/0,03 mmHg.

MS: 168($M^+$, 2), 150(49), 139(68), 121(15), 112(82), 99(100), 81(40), 69(31), 55(30), 43(50).

Spektrale Daten: IR: 3340, 2065, 2955, 2922, 2860, 1650, 1440, 1370, 1300, 1060, 1008, 882, 855 $cm^{-1}$.

Geruch: blumig, fruchtig, blätterartig, schwach bitter, an Kakao erinnernde Nuancen.

*Beispiel 9:*

Analog Beispiel 1 wird das Grignard-Reagens, welches durch Reaktion von 3,38 g (0,139 g-Atome) Magnesium in 20 ml Tetrahydrofuran und 16,5 g (0,139 mol) cis-3-Hexenylchlorid in 70 ml Tetrahydrofuran erhältlich ist, mit 11,8 g (0,12 mol) 2-Methyl-2-pentenal in 20 ml Tetrahydrofuran umgesetzt.

Die fraktionierte Destillation des Rohproduktes (33,1 g) ergibt 12,8 g (58,5%) olfaktisch gutes 4-Methyl-3,8-(Z)-undecadien-5-ol vom Siedepunkt 58° C/0,03 mmHg.

Spektrale Daten: IR: 3340, 3000, 2955, 2922, 2862, 1660, 1452, 1302, 1060, 1000, 855, 720 $cm^{-1}$.

MS: 182($M^+$, 2), 153(13), 100(18), 99(37), 81(28), 71(20), 69(28), 55(28), 43(100), 41(45).

Geruch: grün, nach Butter, blumig.

*Beispiel 10:*

Analog Beispiel 1 wird das Grignard-Reagens, welches durch Reaktion von 7,10 g (0,29 g-Atome) Magnesium in 50 ml Äther und 43,60 g (0,29 mol) 4-Penten-1-ylbromid in 200 ml Äther erhältlich ist, mit 24,50 g (0,25 mol) 2-Methyl-2-pentenal in 50 ml Äther umgesetzt.

Die fraktionierte Destillation des Rohproduktes (43,7 g) ergibt 29,8 g (70,8%) olfaktisch gutes 4-Methyl-3,9-decadien-5-ol vom Siedepunkt 101° C/12 mmHg.

Spektrale Daten: IR: 3340, 3075, 2960, 2925, 2860, 1645, 1460, 1304, 1060, 1015, 990, 908, 853 $cm^{-1}$.

MS: 168($M^+$, 3), 139(30), 125(8), 99(100), 81(37), 71(17), 69(31), 55(28), 43(92), 41(31).

Geruch: grün, metallisch, fettig.

*Beispiel 11:*

Analog Beispiel 1 wird das Grignard-Reagens, welches durch Reaktion um 6,60 g (0,27 g-Atome) Magnesium in 50 ml Äther und 45,00 g (0,28 mol) 4-Methyl-3-penten-1-ylbromid in 150 ml Äther erhältlich ist, mit 23,56 g (0,24 mol) 2-Methyl-2-pentenal in 70 ml Äther umgesetzt.

Die fraktionierte Destillation des Rohproduktes (50,0 g) ergibt 31,7 g (72,4%) olfaktisch gutes 2,7-Dimethyl-2,7-decadien-6-ol (bzw. 4,9-Dimethyl-3,8-decadien-5-ol) vom Siedepunkt 115° C/12 mmHg.

Spektrale Daten: IR: 3350, 2960, 2922, 2865, 2855, 1670, 1450, 1375, 1105, 1052, 1005, 855, 829 $cm^{-1}$.

MS: 182 ($M^+$, 20), 153(53), 135(16), 125(19), 121(14), 99(55), 97(42), 93(25),

83(37), 81(50), 71(35), 69(60), 55(47), 43(100), 41(62).

Geruch: fettig, aldehydisch, grün, blumig.

*Beispiel 12:*

Analog den vorhergehenden Beispielen werden 0,89 g (0,037 g-Atome) Magnesium in 5 ml absolutem Äther bei der Siedetemperatur des Äthers mit einer Lösung von 6,6 g (0,037 mol) 5-Methyl-4-hexenylbromid in 20 ml absolutem Äther zur Reaktion gebracht. Nach beendeter Zugabe wird noch 30 min bei Rückflusstemperatur gehalten. Anschliessend werden bei Raumtemperatur 3,0 g (0,03 mol) 2-Methyl-2-pentenal in 5 ml absolutem Äther während 30 min so zugetropft, dass der Äther wieder zu sieden beginnt. Nach einer weiteren Stunde Rückflussieren wird das Reaktionsgemisch abgekühlt, mit Eis und gesättigter Ammoniumchloridlösung zersetzt und aufgearbeitet. Man erhält so 2,9 g 4,10-Dimethyl-3,9-undecadien-5-ol vom Siedepunkt 110° C/ 0,06 mmHg, $n_D^{20}$ = 1,4705.

Spektrale Daten: IR: 3360, 1670, 1454, 1380, 1072, 1024, 1000, 864 $cm^{-1}$.

MS: 196(6, $M^+$), 167(13), 149(5), 125(26), 99(35), 96(25), 82(100), 81(52), 69(31), 55(37), 43(71).

Geruch: fruchtig (Melone), blumig (Cyclamen), frisch, grün.

*Beispiel 13:*

Grün-blumige Parfümeriebase

| | Gew.-Teile |
|---|---|
| Hydroxycitronellal | 250 |
| Methyldihydrojasmonat | 250 |
| Dipropylenglykol | 200 |
| Bergamottöl | 100 |
| Citronellol | 50 |
| p-Menthan-8-thiol-3-on | 10 |
| Mandarinenöl | 10 |
| Galbanumöl | 10 |
| Jasmin synthetisch | 10 |
| Palmarosaöl | 10 |
| Mastix absolut | 5 |
| Geraniumöl | 5 |
| Cyclamenaldehyd | 5 |
| Korianderöl | 5 |
| Phenyläthylisobutyrat | 5 |
| cis-Hexenol 10% in Dipropylenglykol | 5 |
| Basilikumöl | 3 |
| Cassisknospenöl absolut | 2 |
| | 935 |

Der Zusatz von 65 Teilen 4-Methyl-3-undecen-5-ol bewirkt ein Unterstreichen der blumigen Seite der ursprünglichen Komposition. Es entsteht ein vorher nicht erkennbarer Maiglöckchencharakter.

Durch Zusatz von 65 Teilen 4-Methyl-3,8-(Z)-undecadien-5-ol ändert sich der Geruchscharakter der Komposition in eindrücklicher Weise. Es entsteht ein fruchtiger (nach Beeren), würziger Eindruck, und gleichzeitig wird durch Komplexierung mit den Citrusbestandteilen der Komposition ein Eau-de-Cologne-Effekt bewirkt.

*Beispiel 14:*

Allgemein blumige Base

| | Gew.-Teile |
|---|---|
| Dipropylenglykol | 200 |
| Limonen | 150 |
| α-Jonon | 60 |
| Citronellol | 50 |
| Linalool | 50 |
| Vertofix cœur | 50 |
| Galaxolide 50(1,3,4,6,7,8-Hexahydro-4,6,6,7,8,8-hexamethylcyclopenta-γ-2-benzopyran) | 30 |
| Benzylacetat | 30 |
| 2-Äthyl-3,6,6-trimethyl-2-cyclohexen-1-ylcarbonsäureäthylester | 30 |
| Jasmin synthetisch | 20 |
| Ketonmoschus | 20 |
| Phenyläthyltiglat | 20 |
| 2,28-Trimethyl-7-nonen-3-ol | 15 |
| Weihrauchharz (50% Dipropylenglykol) | 15 |
| Citronellylacetat | 10 |
| Hexenylacetat (10% Dipropylenglykol) | 10 |
| Ylang-Ylang-Öl | 10 |
| Ylang-Ylang synthetisch | 10 |
| Zitronenöl | 10 |
| Undecalakton | 10 |
| Cyclamenaldehyd | 5 |
| Galbanumöl | 5 |
| Sandelholzöl | 5 |
| Jonquille absolut (10% Dipropylenglykol) | 5 |
| *Ciste labdanum* Öl | 5 |
| Adoxal (10% Dipropylenglykol) | 5 |
| | 830 |

170 Teile 4-Methyl-3-dodecen-5-ol unterstreichen in obiger Komposition den sandelblumigen Komplex und verstärken damit den gewünschten Eindruck dieser Komposition.

170 Teile 4,6,8-Trimethyl-3-nonen-5-ol bewirken dagegen einen sehr verblüffenden Effekt in der Base: trotz des sehr grünen Charakters dieser Substanz entsteht in der Base ein sehr angenehm beeriger, auch kosmetisch grün-blumig wirkender Geruchskomplex, der sich gut für Seifen eignet.

Der Zusatz von 2,6-Dimethyl-1,6-nonadien-5-ol bewirkt eine Betonung des grün-blumigen Aspektes, d.h. die neue Verbindung komplexiert mit anderen Komponenten (z.B. mit Galbanum) der Grundbase.

*Beispiel 15:*

Parfümerie (Chypre)-Base

| | Gew.-Teile |
|---|---|
| 1-Methyl-1-methoxycyclododecan | 200 |
| Bergamottöl | 150 |
| Hydroxycitronellal | 100 |
| Citronellol | 80 |
| Petitgrainöl | 60 |
| 12-Oxahexadecanolid | 60 |
| Korianderöl | 40 |
| Galbanumöl | 40 |
| Zedernholzöl | 40 |
| Patchouliöl | 40 |
| Zitronenöl | 40 |
| Elemiöl | 10 |

| | Gew.-Teile |
|---|---|
| Eichenmoos absolut | 10 |
| Fichtennadelöl | 125 |
| | 995 |

Der Zusatz von 5 Teilen 4-Methyl-3-undecen-5-ol bewirkt eine Auffrischung der grünen und krautigen Aspekte dieser Komposition.

5 Teile 4,6,8-Trimethyl-3-nonen-5-ol andererseits bewirken in dieser Base eine viel grössere Diffusion, die neue Base wirkt viel frischer, grüner und auch krautig, so dass sie für ein modernes Herren-Cologne sehr geeignet ist. Der Effekt ist ähnlich mit dem mit 4-Methyl-3-undecen-5-ol erzielten, aber wesentlich ausgeprägter.

*Beispiel 16:*

Parfümeriebase Richtung Fougère

| | Gew.-Teile |
|---|---|
| Lavendelöl | 200 |
| Amylsalicylat | 180 |
| Baummoos 50% in Dipropylenglykol | 100 |
| Citronellol | 100 |
| Geraniol | 80 |
| Ambrettemoschus | 80 |
| Bergamottöl | 80 |
| α-Jonon | 80 |
| α-Amylzimtaldehyd | 25 |
| Eugenol | 25 |
| Metambrate® Giv (1-acetoxy-1-methyl-2-sec.-butylcyclohexan) | 20 |
| | 970 |

Gibt man zu dieser Fougèrekomposition 30 Teile 4-Methyl-3-undecen-5-ol, so wird diese viel frischer und grüner; sie erhält mehr Volumen (wesentlich verstärkte Diffusion) und wirkt dadurch viel stärker als die Komposition ohne Zusatz.

4,6,8-Trimethyl-3-nonen-5-ol wirkt durch ein Unterstreichen speziell der krautigen Note. Der Zusatz vermittelt so der Fougèrekomposition mehr Leben.

*Beispiel 17:*

Parfümeriekomposition Typ Cologne

| | Gew.-Teile |
|---|---|
| Dipropylenglykol | 450 |
| Myrascone® Giv (2-Äthyl-3,6,6,-trimethylcyclohexen-1-ylcarbonsäureäthylester) | 80 |
| Galaxolide 50® | 60 |
| Hydroxycitronellal | 60 |
| Madrox® Giv (1-Methyl-1-methoxycyclododecan) | 60 |
| Sandela | 60 |
| Bergamottöl | 60 |
| Fichtennadelöl | 30 |
| Ketonmoschus | 30 |
| Givescone® Giv | 30 |
| 3,7,7-Trimethyl-3-(3′-methyl-2′-butenyl)bicyclo-[4.1.0]-heptan-4-on (Isomerengemisch) | 20 |
| Petitgrainöl | 15 |
| p-Menthan-8-thiol-3-on | 5 |
| Baummoos absolut | 5 |
| | 965 |

Ein Zusatz von 35 Teilen 4-Methyl-3-undecen-5-ol wirkt durch blumige Nuancierung dieses Eau-de-Cologne-Typs und unterstreicht damit dessen femininen Charakter.

Durch Zusatz von 35 Teilen 2,6-Dimethyl-1,6-nonadien-5-ol wird die Colognebase dagegen in der Folge viel würziger und krautiger. Sie wirkt sehr viel frischer und belebter und tendiert jetzt viel mehr in Richtung Herren-Eau-de-Cologne.

## Patentansprüche

1. Verbindungen der Formel:

$$R^2-\overset{R^1}{\overset{|}{C}}H-\overset{OH}{\overset{|}{C}}H-\overset{CH_3}{\overset{|}{C}}=CH-CH_2-CH_3 \qquad (I)$$

worin $R^1$ für Wasserstoff und $R^2$ für geradkettiges oder endständig einmal verzweigtes $C_{5-7}$-Alkyl oder $C_{4-7}$-Alkenyl steht, oder $R^1$ Methyl und $R^2$ geradkettiges oder endständig einmal verzweigtes $C_{4-7}$-Alkyl ist.

2. 4-Methylundec-3-en-5-ol.

3. 4,6-Dimethyldec-3-en-5-ol.

4. 4,9-Dimethyldec-3-en-5-ol.

5. 4,6,8-Trimethylnon-3-en-5-ol.

6. 2,6-Dimethyl-1,6-nonadien-5-ol.

7. Eine Verbindung ausgewählt aus 4-Methyldodec-3-en-5-ol, 4-Methyltridec-3-en-5-ol, 4,6-Dimethylundec-3-en-5-ol, 4,7,8-Trimethylnon-3-en-5-ol, 4-Methyl-3,9-decadien-5-0l, 4-Methyl-3,8-undecadien-5-ol, 4,9-Dimethyl-3,8-decadien-5-ol, 4,10-Dimethyl-3,9-undecadien-5-ol.

8. Verbindungen der Formel:

$$R^2-\overset{R^1}{\overset{|}{C}}H-\overset{OH}{\overset{|}{C}}H-\overset{CH_3}{\overset{|}{C}}=CH-CH_2-CH_3 \qquad (I)$$

worin $R^1$ für Wasserstoff und $R^2$ für geradkettiges oder endständig einmal verzweigtes $C_{5-7}$-Alkyl oder $C_{4-7}$-Alkenyl steht, oder $R^1$ Methyl und $R^2$ geradkettiges oder endständig einmal verzweigtes $C_{4-7}$-Alkyl darstellt, als Riechstoffe.

9. Riechstoffkomposition, gekennzeichnet durch einen Gehalt an einer Verbindung der Formel:

$$R^2-\overset{R^1}{\overset{|}{C}}H-\overset{OH}{\overset{|}{C}}H-\overset{CH_3}{\overset{|}{C}}=CH-CH_2-CH_3 \qquad (I)$$

worin $R^1$ für Wasserstoff und $R^2$ für geradkettiges oder endständig einmal verzweigtes $C_{5-7}$-Alkyl oder $C_{4-7}$-Alkenyl steht, oder $R^1$ Methyl und $R^2$ geradkettiges oder endständig einmal verzweigtes $C_{4-7}$-Alkyl darstellt.

10. Riechstoffkomposition, gekennzeichnet durch einen Gehalt an 4-Methylundec-3-en-5-ol.

11. Riechstoffkomposition, gekennzeichnet durch einen Gehalt an 4,6-Dimethyldec-3-en-5-ol.

12. Riechstoffkomposition, gekennzeichnet durch einen Gehalt an 4,9-Dimethyldec-3-en-5-ol.

13. Riechstoffkomposition, gekennzeichnet durch einen Gehalt an 4,6,8-Trimethylnon-3-en-5-ol.

14. Riechstoffkomposition, gekennzeichnet durch einen Gehalt an 2,6-Dimethyl-1,6-nonadien-5-ol.

15. Verfahren zur Herstellung von Verbindungen der Formel:

$$R^2-\overset{\overset{R^1}{|}}{C}H-\overset{\overset{OH}{|}}{C}H-\overset{\overset{CH_3}{|}}{C}=CH-CH_2-CH_3 \qquad (I)$$

worin $R^1$ für Wasserstoff und $R^2$ für geradkettiges oder endständig einmal verzweigtes $C_{5-7}$-Alkyl oder $C_{4-7}$-Alkenyl steht, oder $R^1$ Methyl und $R^2$ geradkettiges oder endständig einmal verzweigtes $C_{4-7}$-Alkyl darstellt, dadurch gekennzeichnet, dass man 2-Methyl-2-pentenal mit einer Verbindung der Formel:

$$R^2-\overset{\overset{R^1}{|}}{C}H-MgX \qquad (II)$$

worin $R^1$ und $R^2$ obige Bedeutung besitzen und X für Halogen steht, umsetzt.

16. Verwendung von Verbindungen der Formel:

$$R^2-\overset{\overset{R^1}{|}}{C}H-\overset{\overset{OH}{|}}{C}H-\overset{\overset{CH_3}{|}}{C}=CH-CH_2-CH_3 \qquad (I)$$

worin $R^1$ für Wasserstoff und $R^2$ für geradkettiges oder endständig einmal verzweigtes $C_{5-7}$-Alkyl oder $C_{4-7}$-Alkenyl steht, oder $R^1$ Methyl und $R^2$ geradkettiges oder endständig einmal verzweigtes $C_{4-7}$-Alkyl darstellt, als Riechstoffe.

17. Verwendung von 4-Methylundec-3-en-5-ol als Riechstoff.

18. Verwendung von 4,6-Dimethyldec-3-en-5-ol als Riechstoff.

19. Verwendung von 4,9-Dimethyldec-3-en-5-ol als Riechstoff.

20. Verwendung von 4,6,8-Trimethylnon-3-en-5-ol als Riechstoff.

21. Verwendung von 2,6-Dimethyl-1,6-nonadien-5-ol als Riechstoff.

## Revendications

1. Composés de formule:

$$R^2-\overset{\overset{R^1}{|}}{C}H-\overset{\overset{OH}{|}}{C}H-\overset{\overset{CH_3}{|}}{C}=CH-CH_2-CH_3 \qquad (I)$$

dans laquelle $R^1$ représente l'hydrogène et $R^2$ représente un groupe alkyle en $C_5$-$C_7$ ou alcényle en $C_4$-$C_7$ à chaîne droite ou ramifiée une fois en position terminale, ou bien $R^1$ représente un groupe méthyle et $R^2$ un groupe alkyle en $C_4$-$C_7$ à chaîne droite ou ramifiée une fois en position terminale.

2. Le 4-méthylundéca-3-ène-5-ol.

3. Le 4,6-diméthyldéca-3-ène-5-ol.

4. Le 4,9-diméthyldéca-3-ène-5-ol.

5. Le 4,6,8-triméthylnona-3-ène-5-ol.

6. Le 2,6-diméthyl-1,6-nonadiène-5-ol.

7. Un composé choisi parmi le 4-méthyldodéca-3-ène-5-ol, le 4-méthyltridéca-3-ène-5-ol, le 4,6-diméthylundéca-3-ène-5-ol, le 4,7,8-triméthylnona-3-ène-5-ol, le 4-méthyl-3,9-décadiène-5-ol, le 4-méthyl-3,8-undécadiène-5-ol, le 4,9-diméthyl-3,8-décadiène-5-ol, le 4,10-diméthyl-3,9-undécadiène-5-ol.

8. Composés de formule:

$$R^2-\overset{\overset{R^1}{|}}{C}H-\overset{\overset{OH}{|}}{C}H-\overset{\overset{CH_3}{|}}{C}=CH-CH_2-CH_3 \qquad (I)$$

dans laquelle $R^1$ représente l'hydrogène et $R^2$ un groupe alkyle en $C_5$-$C_7$ ou un alcényle en $C_4$-$C_7$ à chaîne droite ou ramifiée une fois en position terminale, ou bien $R^1$ représente un groupe méthyle et $R^2$ un groupe alkyle en $C_4$-$C_7$ à chaîne droite ou ramifiée une fois en position terminale, en tant que matières odorantes.

9. Composition odorante, caractérisée en ce qu'elle contient un composé de formule:

$$R^2-\overset{\overset{R^1}{|}}{C}H-\overset{\overset{OH}{|}}{C}H-\overset{\overset{CH_3}{|}}{C}=CH-CH_2-CH_3 \qquad (I)$$

dans laquelle $R^1$ représente l'hydrogène et $R^2$ un groupe alkyle en $C_5$-$C_7$ ou alcényle en $C_4$-$C_7$ à chaîne droite ou ramifiée une fois en position terminale, ou bien $R^1$ représente un groupe méthyle et $R^2$ un groupe alkyle en $C_4$-$C_7$ à chaîne droite ou ramifiée une fois en position terminale.

10. Composition odorante, caractérisée en ce qu'elle contient du 4-méthylundéca-3-ène-5-ol.

11. Composition odorante, caractérisée en ce qu'elle contient du 4,6-diméthyldéca-3-ène-5-ol.

12. Composition odorante, caractérisée en ce qu'elle contient du 4,9-diméthyldéca-3-ène-5-ol.

13. Composition odorante, caractérisée en ce qu'elle contient du 4,6,8-triméthylnona-3-ène-5-ol.

14. Composition odorante, caractérisée en ce qu'elle contient du 2,6-diméthyl-1,6-nonadiène-5-ol.

15. Procédé de préparation des composés de formule:

$$R^2-\overset{\overset{R^1}{|}}{C}H-\overset{\overset{OH}{|}}{C}H-\overset{\overset{CH_3}{|}}{C}=CH-CH_2-CH_3 \qquad (I)$$

dans laquelle $R^1$ représente l'hydrogène et $R^2$ un groupe alkyle en $C_5$-$C_7$ ou alcényle en $C_4$-$C_7$ à chaîne droite ou ramifiée une fois en position terminale, ou bien $R^1$ représente un groupe méthyle et $R^2$ un groupe alkyle en $C_4$-$C_7$ à chaîne droite ou ramifiée une fois en position terminale, caractérisé en ce que l'on fait réagir le 2-méthyl-2-penténal avec un composé de formule:

$$R^2-\overset{\overset{R^1}{|}}{C}H-MgX \qquad (II)$$

dans laquelle $R^1$ et $R^2$ ont les significations indiquées ci-dessus et X représente un halogène.

16. Utilisation des composés de formule:

$$\begin{array}{ccccccc} & R^1 & & OH & & CH_3 & \\ & | & & | & & | & \\ R^2- & CH & - & CH & - & C & =CH-CH_2-CH_3 \end{array} \qquad (I)$$

dans laquelle $R^1$ représente l'hydrogène et $R^2$ un groupe alkyle en $C_5$-$C_7$ ou alcényle en $C_4$-$C_7$ à chaîne droite ou ramifiée une fois en position terminale, ou bien $R^1$ représente un groupe méthyle et $R^2$ un groupe alkyle en $C_4$-$C_7$ à chaîne droite ou ramifiée une fois en position terminale, en tant que matières odorantes.

17. Utilisation du 4-méthylundéca-3-ène-5-ol en tant que matière odorante.

18. Utilisation du 4,6-diméthyldéca-3-ène-5-ol en tant que matière odorante.

19. Utilisation du 4,9-diméthyldéca-3-ène-5-ol en tant que matière odorante.

20. Utilisation du 4,6,8-triméthylnona-3-ène-5-ol en tant que matière odorante.

21. Utilisation du 2,6-diméthyl-1,6-nonadiène-5-ol en tant que matière odorante.

## Claims

1. Compounds of the formula:

$$\begin{array}{ccccccc} & R^1 & & OH & & CH_3 & \\ & | & & | & & | & \\ R^2- & CH & - & CH & - & C & =CH-CH_2-CH_3 \end{array} \qquad (I)$$

wherein $R^1$ stands for hydrogen and $R^2$ stands for straight-chain or terminally monobranched $C_{5-7}$-alkyl or $C_{4-7}$-alkenyl, or $R^1$ is methyl and $R^2$ is saturated or terminally monobranched $C_{4-7}$-alkyl.

2. 4-Methyl-undec-3-en-5-ol.

3. 4,6-Dimethyl-dec-3-en-5-ol.

4. 4,9-Dimethyl-dec-3-en-5-ol.

5. 4,6,8-Trimethyl-non-3-en-5-ol.

6. 2,6-Dimethyl-1,6-nonadien-5-ol.

7. A compound selected from 4-methyl-dodec-3-en-5-ol, 4-methyl-tridec-3-en-5-ol, 4,6-dimethyl-undec-3-en-5-ol, 4,7,8-trimethyl-non-3-en-5-ol, 4-methyl-3,9-decadien-5-ol, 4-methyl-3,8-undecadien-5-ol, 4,9-dimethyl-3,8-decadien-5-ol, 4,10-dimethyl-3,9-undecadien-5-ol.

8. Compounds of the formula:

$$\begin{array}{ccccccc} & R^1 & & OH & & CH_3 & \\ & | & & | & & | & \\ R^2- & CH & - & CH & - & C & =CH-CH_2-CH_3 \end{array} \qquad (I)$$

wherein $R^1$ stands for hydrogen and $R^2$ stands for straight-chain or terminally monobranched $C_{5-7}$-alkyl or $C_{4-7}$-alkenyl, or $R^1$ represents methyl and $R^2$ represents straight-chain or terminally monobranched $C_{4-7}$-alkyl, as odorant substances.

9. An odorant substance composition, characterized by a content of a compound of the formula:

$$\begin{array}{ccccccc} & R^1 & & OH & & CH_3 & \\ & | & & | & & | & \\ R^2- & CH & - & CH & - & C & =CH-CH_2-CH_3 \end{array} \qquad (I)$$

wherein $R^1$ stands for hydrogen and $R^2$ stands for straight-chain or terminally monobranched $C_{5-7}$-alkyl or $C_{4-7}$-alkenyl, or $R^1$ represents methyl and $R^2$ represents straight-chain or terminally monobranched $C_{4-7}$-alkyl.

10. An odorant substance composition, characterized by a content of 4-methyl-undec-3-en-5-ol.

11. An odorant substance composition, characterized by a content of 4,6-dimethyl-dec-3-en-5-ol.

12. An odorant substance composition, characterized by a content of 4,9-dimethyl-dec-3-en-5-ol.

13. An odorant substance composition, characterized by a content of 4,6,8-trimethyl-non-3-en-5-ol.

14. An odorant substance composition, characterized by a content of 2,6-dimethyl-1,6-nonadien-5-ol.

15. A process for the manufacture of compounds of the formula:

$$\begin{array}{ccccccc} & R^1 & & OH & & CH_3 & \\ & | & & | & & | & \\ R^2- & CH & - & CH & - & C & =CH-CH_2-CH_3 \end{array} \qquad (I)$$

wherein $R^1$ stands for hydrogen and $R^2$ stands for straight-chain or terminally monobranched $C_{5-7}$-alkyl or $C_{4-7}$-alkenyl, or $R^1$ represents methyl and $R^2$ represents straight-chain or terminally monobranched $C_{4-7}$-alkyl, characterized by reacting 2-methyl-2-pentenal with a compound of the formula:

$$\begin{array}{ccc} & R^1 & \\ & | & \\ R^2- & CH & -MgX \end{array} \qquad (II)$$

wherein $R^1$ and $R^2$ have the above significance and X stands for halogen.

16. The use of compounds of the formula:

$$\begin{array}{ccccccc} & R^1 & & OH & & CH_3 & \\ & | & & | & & | & \\ R^2- & CH & - & CH & - & C & =CH-CH_2-CH_3 \end{array} \qquad (I)$$

wherein $R^1$ stands for hydrogen and $R^2$ stands for straight-chain or terminally monobranched $C_{5-7}$-alkyl or $C_{4-7}$-alkenyl, or $R^1$ represents methyl and $R^2$ represents straight-chain or terminally monobranched $C_{4-7}$-alkyl, as odorant substances.

17. The use of 4-methyl-undec-3-en-5-ol as an odorant substance.

18. The use of 4,6-dimethyl-dec-3-en-5-ol as an odorant substance.

19. The use of 4,9-dimethyl-dec-3-en-5-ol as an odorant substance.

20. The use of 4,6,8-trimethyl-non-3-en-5-ol as an odorant substance.

21. The use of 2,6-dimethyl-1,6-nonadien-5-ol as an odorant substance.